(19) Europäisches Patentamt / European Patent Office / Office européen des brevets

(11) **EP 4 740 968 A1**

(12) **EUROPEAN PATENT APPLICATION**
published in accordance with Art. 153(4) EPC

(43) Date of publication:
**13.05.2026 Bulletin 2026/20**

(21) Application number: 24835441.7

(22) Date of filing: 05.07.2024

(51) International Patent Classification (IPC):
*A61K 51/12* (2006.01)   *A61K 51/06* (2006.01)
*A61K 103/32* (2006.01)   *A61P 35/00* (2006.01)

(86) International application number:
**PCT/CN2024/103920**

(87) International publication number:
**WO 2025/007963 (09.01.2025 Gazette 2025/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 06.07.2023 CN 202310820898

(71) Applicant: **Chengdu Shetai Medical Technology Co., Ltd**
**Chengdu, Sichuan 610000 (CN)**

(72) Inventors:
• **WU, Xiaoyan**
**Chengdu, Sichuan 610000 (CN)**
• **TANG, Weikun**
**Chengdu, Sichuan 610000 (CN)**

• **SHU, Chong**
**Chengdu, Sichuan 610000 (CN)**
• **ZHANG, Shimin**
**Chengdu, Sichuan 610000 (CN)**
• **WANG, Shuqun**
**Chengdu, Sichuan 610000 (CN)**
• **HU, Xixi**
**Chengdu, Sichuan 610000 (CN)**
• **CHEN, Jianxia**
**Chengdu, Sichuan 610000 (CN)**
• **LIANG, Jin**
**Chengdu, Sichuan 610000 (CN)**
• **ZHOU, Dan**
**Chengdu, Sichuan 610000 (CN)**
• **HUANG, Xiaoke**
**Chengdu, Sichuan 610000 (CN)**

(74) Representative: **Cabinet Becker et Associés**
**25, rue Louis le Grand**
**75002 Paris (FR)**

(54) **SUSPENSION CONTAINING RADIOACTIVE MICROSPHERES, AND PREPARATION METHOD THEREFOR AND USE THEREOF**

(57) A suspension containing radioactive microspheres. The radioactive microsphere comprises a resin microsphere and a radionuclide loaded on the resin microsphere, wherein the average particle size of the radioactive microsphere is 25 $\mu$m to 40 $\mu$m. By means of controlling the proportion of non-spherical microspheres in the radioactive microspheres to be within 5%, the distribution density of the microspheres at a tumor part is increased, the effect of killing tumors is improved, and the treatment safety is improved.

A   B   C   D

$R_{min}=R$   ✓      $R_{min}=0<0.75R$   ✗      $R_{min}<0.75R$ ✗      $R=0.71R<0.75R$ ✗

**FIG. 1**

EP 4 740 968 A1

## Description

This application claims the priority of Chinese Patent Application No. 2023108208986 filed on July 6, 2023, the disclosure of which applications are incorporated herein by reference in their entirety.

## Technical Field

The present application relates to the medical field, and specifically to a suspension comprising radioactive microspheres, and preparation methods and uses thereof.

## Background

There are currently many treatments for malignant tumors, including chemotherapy, radiation therapy, interventional therapy, and bio-immunotherapy. Radiation therapy has become a key treatment for certain cancers as most patients have been diagnosed with cancer at an intermediate to advanced stage, losing the opportunity for surgical treatment, and are no longer sensitive to chemotherapeutic drugs after multiple chemotherapy treatments.

Radioactive microsphere therapy involves preparing radioactive materials into microspheres of regular size, and the microspheres are injected into the arterial blood supply of the target organ and emit large doses of high-energy rays to kill tumor tissue. Due to the limited tissue penetration depth of the rays, nearby healthy tissues are spared. Radioactive microspheres mainly comprise a carrier and a nuclide loaded on the carrier, and the nuclides currently being used in radioactive microspheres include yttrium[$^{90}$Y], phosphorus[$^{32}$P], iodine[$^{131}$I], iodine[$^{125}$I], technetium[$^{99m}$Tc], rhenium [$^{99m}$Tc], and holmium[$^{166}$Ho]. Resin microspheres, which have a density closer to that of blood and are thus less likely to settle, have been used as carriers clinically, especially in the treatment of liver cancer (HCC).

Although radioactive microsphere therapy is less harmful to patients' normal tissues, some patients still experience adverse reactions after being subjected to the radioactive microsphere therapy, and the existing radioactive microspheres are more effective against small tumors with a volume of <10 cc, and the distribution of radioactive microspheres within the boundaries of large tumors with a volume of >10 cc, especially in deeper parts of the tumors, needs to be improved.

## Summary

In view of this, the present application provides a suspension comprising radioactive microspheres and preparation methods and uses thereof, aiming to reduce the adverse reactions that the existing radioactive microspheres bring about and improve therapeutic effects.

In a first aspect, the present application provides a suspension comprising radioactive microspheres, wherein the suspension comprises radioactive microspheres; the radioactive microspheres comprise resin microspheres and radionuclides loaded on the resin microspheres; the radioactive microspheres have an average particle size of 25 $\mu$m to 40 $\mu$m; and the radioactive microspheres have a non-spherical ratio of 5% or less. Alternatively, the non-spherical ratio means the ratio of the number of non-spherical radioactive microspheres to the total number of radioactive microspheres in the suspension, and a non-spherical radioactive microsphere is a radioactive microsphere with a minimum radius of less than 75% of the radius of an initial microsphere.

Alternatively, the percentage of radioactive microspheres of 20 $\mu$m to 60 $\mu$m in the suspension is greater than 85% in terms of the number of the radioactive microspheres. Alternatively, the material of the resin microspheres is at least one material selected from the group consisting of crosslinked polystyrene resin, crosslinked polyethylene resin, and crosslinked polydivinylbenzene resin.

Alternatively, the resin microspheres are any one of resin microspheres with sulfonic acid groups, resin microspheres with nitro groups, and carbonized resin microspheres. Alternatively, the radionuclides are selected from an isotope of at least one of yttrium, lutetium, indium, holmium, samarium, iodine, phosphorus, iridium, or rhenium.

Alternatively, the radioactive microspheres of the suspension have a non-spherical ratio of 2% or less.

Alternatively, the material of the resin microspheres is selected from the group consisting of a polystyrene resin with sulfonic acid groups and crosslinked with divinylbenzene. Alternatively, the radionuclides are selected from yttrium [$^{90}$Y].

Alternatively, the suspension comprises yttrium [$^{90}$Y] with an activity of 300-700 MBq per milliliter (1 mL).

Alternatively, the suspension further comprises water for injections.

In a second aspect, the present application provides a suspension comprising radioactive microspheres, wherein the radioactive microspheres comprise resin microspheres and radionuclides loaded on the resin microspheres, the material of the resin microspheres comprises at least one of polystyrene, polyethylene, or polydivinylbenzene, or a crosslinked polymer thereof, and the radioactive microspheres have an average particle size of 25 $\mu$m to 40 $\mu$m, and a non-

spherical ratio of 5% or less. Alternatively, the non-spherical ratio means the ratio of the number of non-spherical radioactive microspheres to the total number of radioactive microspheres in the suspension, and a non-spherical radioactive microsphere is a radioactive microsphere with a minimum radius of less than 75% of the radius of an initial microsphere.

Alternatively, the percentage of radioactive microspheres of 20 μm to 60 μm in the suspension is greater than 85% in terms of the number of the radioactive microspheres. and/or the radioactive microspheres have a non-spherical ratio of 2% or less; and/or the material of the resin microspheres is selected from the group consisting of crosslinked polystyrene microspheres with a crosslinking degree of 2% to 10%.

Alternatively, the resin microspheres are sulfonated, nitrated, or carbonized resin microspheres.

Alternatively, the radionuclides are selected from an isotope of at least one of yttrium, lutetium, indium, holmium, samarium, iodine, phosphorus, iridium, or rhenium.

Alternatively, the radionuclides are selected from yttrium [$^{90}$Y], the material of the resin microspheres is selected from sulfonated polystyrene partially crosslinked with divinylbenzene, the suspension further comprises water for injection, and each mL of the suspension comprises yttrium [$^{90}$Y] with an activity of 300-700 MBq.

Alternatively, the material of the resin microspheres is selected from the group consisting of a polystyrene resin carrying sulfonic acid groups and crosslinked with divinylbenzene.

Alternatively, the suspension further comprises water for injections.

Alternatively, the radionuclides are selected from yttrium [$^{90}$Y], and each mL of the suspension comprises yttrium [$^{90}$Y] with an activity of 300-700 MBq.

In a third aspect, the present application provides a method of preparing a suspension comprising radioactive microspheres, the method comprising: mixing resin microspheres and a radionuclide compound in a solution to react, thereby obtaining the suspension comprising radioactive microspheres; the material of the resin microspheres comprising at least one of polystyrene, polyethylene, or polydivinylbenzene, or a crosslinked polymer thereof, and the radioactive microspheres having an average particle size of 25 μm to 40 μm and having a non-spherical ratio of 5% or less.

Alternatively, the resin microspheres are subjected to sulfonation, nitration or carbonization treatment prior to being mixed with the radionuclide compound.

In a fourth aspect, the present application provides a method of preparing a suspension, comprising: mixing resin microspheres and a radionuclide compound in a solution to react, thereby obtaining the suspension comprising radioactive microspheres; the radioactive microspheres have an average particle size of 25 μm to 40 μm and a non-spherical ratio of 5% or less.

Alternatively, the resin microspheres are subjected to sulfonation, nitration or carbonization treatment prior to being mixed with the radionuclide compound.

In a fifth aspect, the present application provides use of the suspension as described in the first aspect or the suspension prepared by the method as described in the second aspect in the preparation of a medicament for treating a tumor.

Alternatively, the tumor is primary or secondary liver cancer.

In a sixth aspect, the present application provides a method of treating a tumor, comprising: administering to a patient with a tumor the suspension described in the first aspect or the suspension prepared by the method described in the second aspect.

Beneficial effects:

The present application provides a suspension comprising radioactive microspheres; the radioactive microspheres comprise resin microspheres and radionuclides loaded on the resin microspheres, and the radioactive microspheres have an average particle size of 25 μm to 40 μm. Controlling the non-spherical ratio of the radioactive microspheres to less than 5% enhances the distribution density of the microspheres in the tumor portion, improves therapeutic efficacy, lowers the incidence of adverse reactions, and improves safety.

**The Description of Drawings**

In order to more clearly illustrate the technical solutions in the embodiments of the present application, the accompanying drawings to be used in the description of the embodiments will be briefly described below.

FIG. 1 shows a schematic diagram of the relationship between the minimum radius ($R_{min}$) of the microsphere and the radius (R) of the initial microsphere in embodiments of the present application, with A in FIG. 1 being an example of a spherical microsphere, and B, C, and D each representing an embodiment of a non-spherical microsphere, respectively.

FIG. 2 shows a microscopic photograph of embodiments of the present application comprising non-spherical radioactive polystyrene microspheres.

FIG. 3 shows a schematic diagram of the implantation position of a test sample or a control sample in embodiments of the present application.

## The detailed description of embodiments

In order to make the technical problems, technical solutions and technical effects to be solved by the present disclosure more clear and understandable, the present disclosure will be described in further detail in the following in connection with specific embodiments, and it is obvious that the described embodiments are only a part of the embodiments of the present invention and not all of the embodiments. Based on the embodiments in the present invention, all other embodiments obtained by those skilled in the art without paying for creative labor fall within the scope of protection of the present application.

It has been found that the radioactive microspheres in the prior art have defects of producing adverse reactions during the treatment process and the therapeutic efficacy needs improvement, such as radioactive polystyrene microspheres prepared from commercially available sulfonated polystyrene microspheres, or radioactive polystyrene microspheres or carbon microspheres prepared using the methods provided in CN202211592412X, CN2022116010239, and CN2022116017505.

The inventors unexpectedly discovered that the non-spherical ratio of the radioactive microspheres significantly affects their therapeutic efficacy as well as their adverse reactions in subjects after implantation. The non-spherical ratio of microspheres was found to be one of the key factors affecting their distribution density in the body, which ultimately affects efficacy and safety. When the non-spherical ratio of the suspension comprising radioactive microspheres is controlled within 5%, it not only increases the distribution density of microspheres within the tumor, indicating a better killing effect on the tumor, but also significantly reduces irritant and inflammatory responses, thereby reducing the incidence of adverse reactions such as fever, pain and impaired liver function. The radioactive microspheres ultimately produced by the prior art have a large non-spherical ratio, which is basically greater than 10%. Even though the possibility of non-spherical microsphere generation during the sulfonation process was relatively reduced by controlling the stirring speed, the non- spherical ratio of the radioactive microspheres finally produced was still greater than 10%. The prior art did not recognize the importance of the non-spherical ratio of suspensions comprising radioactive microspheres.

In view of the foregoing, embodiments of the present application first provide a suspension comprising radioactive microspheres, and the suspension comprising radioactive microspheres is used for radiotherapy directed to malignant tumors. The radioactive microspheres comprise resin microspheres and radionuclides loaded on the resin microspheres, the radioactive microspheres have an average particle size of 25 $\mu$m to 40 $\mu$m (micrometers), and the radioactive microspheres have a non-spherical ratio of 5% or less. Compared to the prior art, controlling the non-spherical ratio of the radioactive microspheres within such a range not only improves the therapeutic effect but also enhances safety.

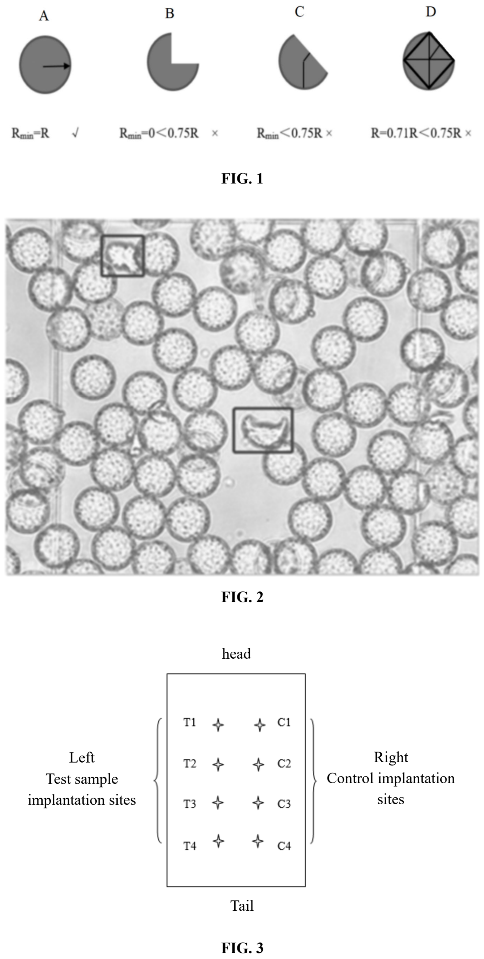

The term "non-spherical ratio" herein refers to the ratio of the number of non-spherical microspheres to the total number of microspheres in a suspension. Non-spherical microspheres are microspheres whose minimum radius is less than 75% of the radius of the initial microsphere. Accordingly, "spherical ratio" refers to the ratio of spherical microspheres to the total number of microspheres in a given amount of suspension, and the spherical microspheres are those with a minimum radius ($R_{min}$) higher than 75% of the initial microsphere radius (R). $R_{min}$ refers to the shortest distance measured from the center of a circle to its edge when viewed in orthographic projection., and the center of the circle is the center of the two points on the microsphere that have the longest straight-line distances in orthographic projection (if the center of the circle is equidistant from any point on the edge of the microsphere, it is not counted as a non-spherical microsphere); R is the average radius of the microspheres, the average radius is relative to the average diameter, the average diameter is the average particle size of all the microspheres in the suspension, and the average radius is half of the average particle size; it will be appreciated that the initial microsphere is a standard round microsphere having a diameter of that average particle size. As shown in FIG. 1, FIG. 1 shows a schematic diagram of the relationship between the minimum radius ($R_{min}$) of the microsphere and the radius (R) of the initial microsphere, in which A in FIG. 1 is an example of a spherical microsphere where $R_{min}$ is equal to R. B, C, and D each represent an example of a non-spherical microsphere, i.e., $R_{min}$ is less than 0.75 times R. It should also be noted that the terms "spherical ratio" and "average sphericity" herein have different meanings and are calculated differently, so that the values of spherical ratio and average sphericity for the same microsphere are different.

The non-spherical ratio may be any value of 5% or less, such as 0 to 5%, 0.1% to 5%, 1% to 4%, 2% to 3%, and the like; and such as, 5% or less, 4% or less, 3% or less, 2% or less, 1% or less, 0.5% or less, 0.4% or less, 0.3% or less, 0.2% or less, 0.1% or less, 0%, and the like.

The average particle size of the radioactive microspheres may be any value in the range of 25 $\mu$m to 40 $\mu$m, such as 30 $\mu$m to 35 $\mu$m, in which range it is favorable for the distribution of the microspheres in the target organ, to improve therapeutic efficacy and reduce side effects. It will be appreciated that the average particle size may be of any value in the range of 25 $\mu$m to 40 $\mu$m, such as 25 $\mu$m, 28 $\mu$m, 30 $\mu$m, 32.5 $\mu$m, 35 $\mu$m, 40 $\mu$m and the like.

In some embodiments, the percentage of radioactive microspheres of 20 $\mu$m to 60 $\mu$m in the suspension is greater than 85%, for example 90% or more, for example again 95% or more. In this range, it is favorable for the distribution of the microspheres in the target organ, helping to improve therapeutic efficacy and reduce side effects. It will be appreciated that the percentage of radioactive microspheres of 20 $\mu$m to 60 $\mu$m may be any value greater than 85% or more, such as 85%, 88%, 90%, 95%, 98%, and the like. It should be noted that the percentage of radioactive microspheres of 20 $\mu$m to 60 $\mu$m refers to the ratio of the number of microspheres with a particle size in that range to the total number of microspheres, wherein the total number of microspheres includes the number of both spherical microspheres as well as non-spherical microspheres. Thus, microspheres in the range of 20 $\mu$m to 60 $\mu$m include spherical microspheres as well as non-spherical microspheres, and the microspheres outside of this range also include both spherical and non-spherical microspheres.

In some embodiments, for better loading of radionuclides, the resin microspheres are selected from, but not limited to, sulfonated, nitrated, and carbonized resin microspheres. When the resin microspheres are sulfonated or nitrated resin microspheres, their surface carry sulfonic acid groups or nitro groups, and the radionuclides bind to sulfonic acid groups or nitro groups and are mostly distributed on the surface of the microspheres. When the resin microspheres are carbonized resin microspheres, they are specifically carbon microspheres. The surface of carbon microspheres comprises mesopores, and radionuclides distribute within these mesopores in the form of complexes. Due to the non-degradable nature of carbon microspheres, non-spherical carbon microspheres induce inflammatory responses to a greater degree. Therefore, controlling the non-spherical particles of carbon microspheres to less than 5% is more effective in alleviating irritation and inflammatory reactions, as well as in reducing the incidence of adverse reactions, than microspheres made of other materials.

In some embodiments, the material of the resin microspheres is selected from, but not limited to, at least one of polystyrene, polyethylene, or polydivinylbenzene, or a crosslinked polymer thereof, and in some embodiments, the material of the resin microspheres is selected from, but not limited to, phenolic resin microspheres. In some embodiments, the resin microspheres are crosslinked polystyrene microspheres with a crosslinking degree of 2% to 10%, such as 4%, 6%, 8%. The requirements for both microsphere strength and loading rate can be met in this range. In some embodiments, the resin microspheres are selected from the group consisting of sulfonated and nitrified cross-linked polystyrene. In some embodiments, the material of the resin microspheres is selected from polystyrene partially crosslinked with divinylbenzene. In some embodiments, the resin microspheres are selected from sulfonated polystyrene partially crosslinked with divinylbenzene.

In some embodiments, the radionuclides are an isotope of at least one of yttrium, lutetium, indium, holmium, samarium, iodine, phosphorus, iridium, or rhenium. In some embodiments, the radionuclide is yttrium [$^{90}$Y].

In some embodiments, the radionuclide is yttrium [$^{90}$Y], the material of the resin microspheres is selected from sulfonated polystyrene partially crosslinked with divinylbenzene, and the suspension further comprises water for injection. In some embodiments, the activity of yttrium [$^{90}$Y] is 300 to 700 MBq, preferably 500 to 700 MBq. In some embodiments, the suspension comprises 2 to 3 mg of resin microspheres per 1 mL. In some embodiments, the suspension further comprises radionuclide compounds, such as yttrium chloride and/or yttrium sulfate. In some embodiments, the suspension further comprises sulfate, wherein a small amount of sulfate is present in the aqueous solution. In some embodiments, the sulfate is sodium sulfate. In some embodiments, the suspension further comprises a buffer, such as phosphate buffer. The embodiments of the present application also provide a suspension comprising radioactive carbon microspheres, wherein the radioactive carbon microspheres comprise carbon microspheres and radionuclides loaded on the carbon microspheres, the radioactive carbon microspheres have an average particle size of 25 $\mu$m to 40 $\mu$m (micrometers), and the radioactive microspheres have a non-spherical ratio of 5% or less.

Accordingly, the embodiments of the present application also provide a method of preparing a suspension comprising radioactive microspheres, comprising: mixing resin microspheres and a radionuclide compound in a solution to react, thereby obtaining the suspension comprising radioactive microspheres; the radioactive microspheres have an average particle size of 25 $\mu$m to 40 $\mu$m and a non-spherical ratio of 5% or less.

In some embodiments, the resin microspheres are further subjected to sulfonation, nitration or carbonization treatment prior to being mixed with the radionuclide compound.

When the resin microspheres are subjected to sulfonation treatment, in some embodiments, the method of preparing a suspension comprising radioactive microspheres comprises the following steps:

> S 10. The resin microspheres are placed in concentrated sulfuric acid for a sulfonation reaction to obtain sulfonated resin microspheres.
> S20. The sulfonated microspheres and a radionuclide compound are mixed in a solution to react, thereby obtaining a suspension comprising radioactive microspheres.

In some embodiments, in step S10, the sulfonation reaction is carried out under an inert atmosphere or nitrogen, such as nitrogen or argon. In this way, the effect of oxidation reaction on the morphology of microspheres during the preparation process can be prevented, which is conducive to controlling the non-spherical ratio of radioactive microspheres.

In some embodiments, in step S10, the temperature of the sulfonation reaction is 35 to 80°C (degrees Celsius), for example: 35°C, 40°C, 50°C, 60°C, 70°C, or 80°C. The duration is from 3 to 6h (hours), such as 4h, 5h or 6h. On the basis of the above embodiments, in some embodiments, the concentrated sulfuric acid is added in batches during the sulfonation reaction process, and the specific scheme is as follows: firstly, the resin microspheres are mixed with a small amount of concentrated sulfuric acid, and the temperature is slowly heated up to a specific temperature at a temperature increase rate of 1~3°C/min (degrees Celsius/min) under stirring, and then the remaining concentrated sulfuric acid is added for sulfonation. In some embodiments, during mixing of the microspheres with a small amount of concentrated sulfuric acid, the ratio of the mass of the resin microspheres to the volume of the concentrated sulfuric acid is 1 g: (1.5~2) mL, and after adding the remaining concentrated sulfuric acid, the ratio of the mass of the resin microspheres to the volume of the concentrated sulfuric acid is 1 g: (3~6) mL. In this way, by using the method of adding concentrated sulfuric acid in batches, not only can there be a better sulfonation effect, but also can improve the problem of excessive heat affecting the morphology of the microspheres during the sulfonation reaction process, so as to control the non-spherical ratio of the radioactive microspheres.

In some embodiments, in step S10, the sulfonation reaction is carried out under an inert atmosphere or nitrogen, and the concentrated sulfuric acid during the sulfonation reaction is added several times. For example, 20%-70% of the amount of concentrated sulfuric acid, such as 30%-68%, 33%-50%, or 50%-68%, is added at initial conditions, and the remaining concentrated sulfuric acid is added after the reaction temperature has risen to a predetermined temperature. Under this condition it is favorable to control the non-spherical ratio of radioactive microspheres of 5% or less.

In some embodiments, in step S20, the radionuclide compound is yttrium chloride, and the concentration of the yttrium chloride solution is from 1 to 3 mg/mL, e.g., 1 mg/mL, 1.5 mg/mL, 2 mg/mL, 2.5 mg/mL, 3 mg/mL, and the like. In some embodiments, the yttrium chloride solution is a mixture comprising yttrium [$^{90}$Y] chloride and yttrium chloride, wherein the amount of yttrium [$^{90}$Y] chloride is determined based on the desired radioactivity.

In some embodiments, the sulfonated, nitrified, or carbonized resin microspheres are reacted with the radionuclide compound at a reaction temperature of 25 to 40°C, e.g., 25°C, 30°C, 35°C, 40°C, and for a time of 1 to 2h, e.g., 1h, 1.5h, 1.8h, 2h.

When the resin microspheres are subjected to carbonization treatment, in some embodiments, the method of preparing a suspension comprising radioactive microspheres comprises the following steps:

S100. The resin microspheres are calcined under an inert or nitrogen atmosphere to produce carbon microspheres. S200. The carbonization microspheres and a radionuclide compound are mixed in a solution to react, thereby obtaining a suspension comprising radioactive microspheres. In some embodiments, the calcination temperature for the microspheres in step S100 is 400 to 800°C, e.g., 400°C, 500°C, 600°C, 700°C, 800°C, and the like, especially 400 to 600°C; the duration is 3 to 6h, for example: 3h, 3.5h, 4h, 4.5h, 5h, 6h, and the like, especially 3 to 4h. On the basis of the above embodiments, in some embodiments, the heating process of calcination is a stepwise heating, and the specific scheme is as follows: firstly, the temperature is rapidly elevated to 150°C to 200°C from room temperature at a rate of 4 to 12°C/min, then the temperature is slowly elevated to a specific calcination temperature from above 150°C to 200°C at a rate of 1 to 2 °C/min (Celsius degrees/min), for calcination. In another specific embodiment, the temperature is first rapidly elevated from room temperature to 150°C to 200°C, then slowly elevated from above 150°C to 200°C at a rate of 1.6 to 2°C/min to 300°C, and then slowly elevated at a rate of 1 to 1.5°C/min to a specific calcination temperature. In this way, the utilization of stepwise heating is not only conducive to improving the production efficiency but also can control the non-spherical ratio of the radioactive microspheres.

In some embodiments, in step S100, the calcined carbon microspheres also undergo a cleaning step and a drying step. For example, the cleaning can be done with dilute nitric acid and deionized water. The drying step can be vacuum drying or drying using a blast drying oven.

In some embodiments, in order to achieve better complexation, a precipitant is further added before or after mixing the carbon microspheres and the radionuclide compound in solution in step S200. For example, in some specific embodiments, the method of preparing the radioactive carbon microspheres comprises any one of Method I or Method II below:

Method I: Mixing carbon microspheres and a solution comprising a radionuclide compound, then adding a solution comprising a precipitating agent for reacting, to get radioactive carbon microspheres;
Method II: Mixing carbon microspheres and a solution comprising a precipitating agent, then adding a solution comprising radionuclide compounds to for reacting, to get radioactive carbon microspheres.

In this way, radionuclides can be generated and grown in the mesopores of the carbon microspheres, thus obtaining radioactive carbon microspheres with a high degree of loading firmness. On this basis, the inventors have found that too rapid or excessive generation of radionuclides in the mesopores of the carbon microspheres can cause the mesopore structure to collapse, resulting in a condition in which the carbon microspheres have an increased non-spherical ratio. Thus, the rate and amount of radionuclide generated in the mesopore is controlled by controlling the concentration of precipitating agent and radionuclides. In view of this, in some embodiments, the concentration of the precipitating agent is 5 mg/mL to 10 mg/mL (milligrams per milliliter), for example 5mg/mL, 6mg/mL, 8mg/mL, 10mg/mL, and the like. In some embodiments, the concentration of the radionuclides is 3 mg/mL to 6 mg/mL, for example 3 mg/mL, 5 mg/mL, 6 mg/mL, and the like Controlling the precipitating agent and radionuclides in the range not only satisfies the radionuclide loading but also slows down the radionuclide generation rate and generation amount in the mesopore, which is favorable for controlling the non-spherical ratio of the carbon microspheres.

In some embodiments, in step S100, the microsphere carbonization process begins with a rapid heating from room temperature at 4 to 12°C/min to 150°C to 200°C, followed by a slow heating at 1 to 2°C/min to a temperature of 400 to 600°C, and calcination for 3 to 4h; in step S200, the concentration of the precipitating agent is from 5 mg/mL to 10 mg/mL and the concentration of the radionuclides is from 3 mg/mL to 6 mg/mL. In this way, it is favorable to control the non-spherical ratio of carbon microspheres of 5% or less.

In some embodiments, the radionuclide compound is selected from, but not limited to, yttrium [$^{90}$Y] sulfate or yttrium [$^{90}$Y] chloride. The precipitating agent is selected from, but not limited to, at least one, such as one or two, of tartaric acid, EDTA, and sodium phosphate. For example:

When there is one precipitating agent, for example EDTA, and the radionuclide compound is yttrium [$^{90}$Y] chloride, for example, the method of preparing the radioactive carbon microspheres comprises: after cleaning, mixing the carbon microspheres with a $^{90}$YCl$_3$ solution homogeneously and then allowing the mixture to stand for 12h, then adding EDTA to the mixture, shaking them in a shaker for 1h, and washing and drying them to produce the $^{90}$Y-EDTA complex loaded carbon microspheres.

When there is one precipitating agent, for example tartaric acid, and the radionuclide compound is yttrium [$^{90}$Y] chloride, exemplarily, the method of preparing the carbon microspheres comprises: after cleaning, mixing the carbon microspheres with a $^{90}$YCl$_3$ solution homogeneously and then allowing the mixture to stand for 12h, then adding tartaric acid to the mixture, shaking them in a shaker for 1h, and washing and drying them to produce the $^{90}$Y- tartaric acid complex loaded carbon microspheres.

When there are two precipitating agents, for example tartaric acid and sodium phosphate, and the radionuclide compound is yttrium [$^{90}$Y] chloride, exemplarily, the method of preparing the carbon microspheres comprises: after cleaning, mixing the carbon microspheres with a $^{90}$YCl$_3$ solution homogeneously and then allowing the mixture to stand for 12h, then adding tartaric acid to the mixture, shaking them in a shaker for 1h, and washing and drying them to produce the $^{90}$Y- tartaric acid complex loaded carbon microspheres; then, adding the $^{90}$Y- tartaric acid complex loaded carbon microspheres into sodium phosphate solution, shaking them in a shaker for 1h, and washing and drying them to produce $^{90}$Y-PO$_4$ complex loaded carbon microspheres.

In some embodiments, the solvents for both the solution of the resin microspheres and the radionuclide compound, and the precipitating agent solution are water for injection. The embodiments of the present application also provide a method of preparing a suspension comprising radioactive carbon microspheres, the method comprising: mixing the carbon microspheres and a radionuclide compound in a solution to react, thereby obtaining the suspension comprising radioactive carbon microspheres; the radioactive carbon microspheres have an average particle size of 25 μm to 40 μm and a non-spherical ratio of 5% or less.

Accordingly, the present application also provides use of the above suspensions in the preparation of a medicament for treating a tumor.

In some embodiments, the tumor is a vascularized solid tumor, and in some embodiments, the tumor is primary or secondary liver cancer.

Further, the present application also provides a method of treating a tumor comprising administering the suspension above to a tumor patient. The above suspensions have better homogeneity due to the presence of radioactive microspheres with lower non-spherical ratio, resulting in better tumor distribution density and significantly improved efficacy in treatment, as well as a better safety profile with a reduced incidence of adverse reactions.

In some embodiments, the method of treatment comprises delivering the suspension to the tumor site, specifically, the suspension may be administered to an arterial blood supply of the target organ to achieve radiotherapy to the tumor site of the target organ. For example, treatment of a liver tumor may be carried out by insertion of a catheter into the hepatic artery, through which the suspension is then delivered to the liver tumor. The suspension may be administered in a single dose or multiple doses until the desired radiation level is achieved.

The technical solutions of the present disclosure will be set forth more clearly and explicitly below by way of illustration in conjunction with Examples. It should be understood that these examples are used for illustrative purposes only and are in no way intended to limit the scope of protection of the present disclosure. The scope of protection of the present disclosure is limited only by the claims.

Unless otherwise noted, the reagents and instruments employed in the following examples are conventional products that can be obtained through commercially available purchases. Unless otherwise stated, experiments were conducted under conventional conditions or those recommended by the manufacturer. In the following examples, the polystyrene microspheres used in the sulfonation treatment are crosslinked polystyrene-divinylbenzene polymer microspheres with a crosslinking degree in a range to 10% and an average particle size of 30 μm (AmberChromTM XT 30 commercially available from DuPont), and the polystyrene microspheres used in the carbonization treatment are crosslinked polystyrene-divinylbenzene polymer microspheres with a crosslinking degree in a range of 2% to 10% and an average particle size of 50 μm (commercially available from Qingdao Hongpu Bio-technology Co.)(both of them are hereinafter referred to as "polystyrene microspheres"). Sulfonated polystyrene microspheres were selected as cation exchange resin Aminex 50W-X4 with an average particle size of 30 μm (Biorad, Hercules, CA). Yttrium [$^{90}$Y] chloride and yttrium [$^{90}$Y] sulfate were obtained by strontium-90 decay. The activity of the radiotracer was measured using a Capintec (CRC-55TW) dose calibrator (USA).

**I. Preparation Example**

**Example 1**

This example provides a suspension comprising radioactive polystyrene microspheres, prepared through the following steps:

(1) After the polystyrene microspheres were washed with absolute ethanol and dried, the polystyrene microspheres were ultrasonically dispersed in a small amount of concentrated sulfuric acid (1 g: 2 mL) under an Ar atmosphere, heated to 75°C at a rate of 1°C/min under stirring at 300 rpm, then added with the remaining concentrated sulfuric acid (added to 1 g: 6 mL), and reacted for 6 h to obtain sulfonated polystyrene microspheres, which were washed and dried.
(2) A solution of yttrium chloride (a mixed solution of yttrium chloride [$^{90}$Y] and yttrium chloride in total concentration of 2 mg/mL) with an activity of 5 GBq was uniformly mixed with 2 g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

**Example 2**

This example provides a suspension comprising radioactive polystyrene microspheres, prepared through the following steps:

(1) After the polystyrene microspheres were washed with absolute ethanol and dried, the polystyrene microspheres were ultrasonically dispersed in a small amount of concentrated sulfuric acid (1 g: 2 mL) under an Ar atmosphere, heated to 75°C at a rate of 2°C/min under stirring at 300 rpm, then added with the remaining concentrated sulfuric acid (added to 1 g: 6 mL), and reacted for 6 h to obtain sulfonated polystyrene microspheres, which were washed and dried.
(2) A solution of yttrium chloride (a mixed solution of yttrium chloride [$^{90}$Y] and yttrium chloride in total concentration of 2.25mg/mL) with an activity of 5 GBq was uniformly mixed with 2g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

**Example 3**

This example provides a suspension comprising radioactive polystyrene microspheres, prepared through the following steps:

(1) After the polystyrene microspheres were washed with absolute ethanol and dried, the polystyrene microspheres were ultrasonically dispersed in a small amount of concentrated sulfuric acid (1 g: 2 mL), and then heated to 75°C at a rate of 2°C/min under stirring conditions of 300 rpm, then added with the remaining concentrated sulfuric acid (added to 1 g: 6 mL), and reacted for 6 h to obtain sulfonated polystyrene microspheres, which were washed and dried.
(2) A solution of yttrium chloride (a mixed solution of yttrium chloride [$^{90}$Y] and yttrium chloride in total concentration of 2.25mg/mL) with an activity of 5 GBq was uniformly mixed with 2 g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

**Example 4**

This example provides a suspension comprising radioactive polystyrene microspheres, prepared through the following steps:

(1) After the polystyrene microspheres were washed with absolute ethanol and dried, the polystyrene microspheres were ultrasonically dispersed in a small amount of concentrated sulfuric acid (1 g: 1.5 mL) under an nitrogen atmosphere, heated to 80°C at a rate of 3°C/min under stirring conditions of 300 rpm, then added with the remaining concentrated sulfuric acid (added to 1 g: 3 mL), and reacted for 6 h to obtain sulfonated polystyrene microspheres, which were washed and dried.
(2) A solution of yttrium chloride (a mixed solution of yttrium chloride [90Y] and yttrium chloride in total concentration of 1mg/mL) with an activity of 5 GBq was uniformly mixed with 2 g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

**Example 5**

This example provides a suspension comprising radioactive polystyrene microspheres, prepared through the following steps:

(1) After the polystyrene microspheres were washed with absolute ethanol and dried, the polystyrene microspheres were ultrasonically dispersed in a small amount of concentrated sulfuric acid (1 g: 2 mL) under an Ar atmosphere, then heated to 80°C at a rate of 1°C/min under stirring conditions of 300 rpm, then added with the remaining concentrated sulfuric acid (added to 1 g: 3 mL), and reacted for 3 h to obtain sulfonated polystyrene microspheres, which were washed and dried.
(2) A solution of yttrium chloride (a mixed solution of yttrium chloride [90Y] and yttrium chloride in total concentration of 3mg/mL) with an activity of 5 GBq was uniformly mixed with 2 g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

**Example 6**

This example provides a suspension comprising radioactive carbon microspheres, prepared through the following steps:

(1) Carbon microspheres were made by calcining polystyrene microspheres under Ar atmosphere at 400°C for 4h (from room temperature to 200°C at 10°C/min, then to 300°C at 2°C/min, and then to 400°C at 1°C/min, held at 400°C for 4h, and then cooled down at the rate of 10°C/min), and then washed with 0.3 mol/L dilute nitric acid, deionized water in sequence for 2 times, followed by drying and sieving treatment.
(2) The carbon microspheres (2g) were mixed with tartaric acid solution (5mg/mL) respectively and left to stand for 12h, then 5GBq of yttrium chloride solution (a mixed solution of yttrium [90Y] chloride and yttrium chloride, 3mg/mL) was added to the mixture and shaken in a constant temperature shaker at 25°C for 1h (the total volume of the solution was 20mL), to obtain the suspension comprising the radioactive carbon microspheres.

**Example 7**

This example provides a suspension comprising radioactive carbon microspheres, prepared through the following steps:

(1) Carbon microspheres were made by calcining polystyrene microspheres under Ar atmosphere at 400°C for 4h (from room temperature to 200°C at 10°C/min, then to 400°C at 2°C/min, and kept for 4h, then cooled down at the rate of 10°C/min), and then washed with 0.3 mol/L dilute nitric acid, deionized water in sequence for 2 times, followed by drying and sieving treatment.
(2) The carbon microspheres (2g) were mixed with tartaric acid solution (10mg/mL) respectively and left to stand for 12h, then 5GBq of yttrium chloride solution (a mixed solution of yttrium [90Y] chloride and yttrium chloride, 6mg/mL) was added to the mixture and shaken in a constant temperature shaker at 25°C for 1h (the total volume of the solution was 20mL), to obtain the suspension comprising the radioactive carbon microspheres.

**Example 8**

This example provides a suspension comprising radioactive carbon microspheres, prepared through the following steps:

(1) Carbon microspheres were made by calcining polystyrene microspheres under Ar atmosphere at 400°C for 4h (from room temperature to 200°C at 10°C/min, then to 400°C at 2°C/min, and kept for 4h, then cooled down at the rate of 10°C/min), and then washed with 0.3 mol/L dilute nitric acid, deionized water in sequence for 2 times, followed by drying and sieving treatment.

(2)The carbon microspheres (2g) were mixed with tartaric acid solution (25mg/mL) respectively and left to stand for 12h, then 5GBq of yttrium chloride solution (a mixed solution of yttrium [90Y] chloride and yttrium chloride, 12.5mg/mL) was added to the mixture and shaken in a constant temperature shaker at 25°C for 1h (the total volume of the solution was 20mL), to obtain the suspension comprising the radioactive carbon microspheres.

**Example 9**

This example provides a suspension comprising radioactive carbon microspheres, prepared through the following steps:

(1) Carbon microspheres were made by calcining polystyrene microspheres under Ar atmosphere at 600°C for 3h (from room temperature to 150°C at 10°C/min, then to 600°C at 2°C/min, and kept for 3h, then cooled down at the rate of 10°C/min), and then washed with 0.3 mol/L dilute nitric acid, deionized water in sequence for 2 times, followed by drying and sieving treatment.

(2) The carbon microspheres (2g) were mixed with tartaric acid solution (10mg/mL) respectively and left to stand for 12h, then 5GBq of yttrium chloride solution (a mixed solution of yttrium [90Y] chloride and yttrium chloride, 5mg/mL) was added to the mixture and shaken in a constant temperature shaker at 25°C for 1h (the total volume of the solution was 20mL), to obtain the suspension comprising the radioactive carbon microspheres.

**Example 10**

This example provides a suspension comprising radioactive carbon microspheres, prepared through the following steps:

(1) Carbon microspheres were made by calcining polystyrene microspheres under Ar atmosphere at 500°C for 3h (from room temperature to 200°C at 10°C/min, then to 500°C at 2°C/min, and kept for 3h, then cooled down at the rate of 10°C/min), and then washed with 0.3 mol/L dilute nitric acid, deionized water in sequence for 2 times, followed by drying and sieving treatment.

(2) The carbon microspheres (2g) were mixed with 5GBq of yttrium chloride solution (a mixed solution of yttrium [90Y] chloride and yttrium chloride, 4mg/mL) respectively and left to stand for 12h, then tartaric acid solution (8mg/mL) was added to the mixture and shaken in a constant temperature shaker at 25°C for 1h (the total volume of the solution was 20mL), to obtain the suspension comprising the radioactive carbon microspheres.

**Comparative Example 1**

This example provides a suspension comprising radioactive polystyrene microspheres, prepared through the following steps:

(1) After the polystyrene microspheres were washed with absolute ethanol and dried, the polystyrene microspheres were ultrasonically dispersed in an appropriate amount of concentrated sulfuric acid (1 g: 6 mL). The mixture was stirred at 300 rpm and reacted at 75 °C for 6 h, to obtain the sulfonated polystyrene microspheres, which were washed and dried.

(2) A solution of yttrium chloride (a mixed solution of yttrium chloride [90Y] and yttrium chloride in total concentration of 2.25mg/mL) with an activity of 5 GBq was uniformly mixed with 2 g of sulfonated polystyrene microspheres by stirring, the mixture was subjected to a load reaction at 40°C for 2 h, and the loaded polystyrene microspheres were washed, and resuspended with 10 mL water to obtain the suspension comprising radioactive polystyrene microspheres.

## Comparative Example 2

This comparative example provides a suspension comprising radioactive polystyrene microspheres, prepared through the following steps:

(1) 2 g of commercially available sulfonated polystyrene microspheres were added into water, followed by reaction with 5 GBq of an yttrium sulfate solution (a mixed solution of yttrium chloride [$^{90}$Y] and yttrium chloride in a total concentration of 2.25 mg/mL) at 40 °C for 2 h.
(2) The microspheres were washed with phosphate buffer solution and then resuspended with 10 mL of water to obtain a suspension comprising radioactive polystyrene microspheres.

## Comparative Example 3

This comparative example provides a suspension comprising radioactive carbon microspheres, prepared through the following steps:

(1) Carbon microspheres were made by calcining polystyrene microspheres under Ar atmosphere at 400°C for 4h (from room temperature to 200°C at 10°C/min, then to 400°C at 3°C/min, and kept for 4h, then cooled down at the rate of 10°C/min), and then washed with 0.3 mol/L dilute nitric acid, deionized water in sequence for 2 times, followed by drying and sieving treatment.
(2) The carbon microspheres (2g) were mixed with tartaric acid solution (25mg/mL) respectively and left to stand for 12h, then 5GBq of yttrium chloride solution (a mixed solution of yttrium [$^{90}$Y] chloride and yttrium chloride, 12.5mg/mL) was added to the mixture and shaken in a constant temperature shaker at 25°C for 1h (the total volume of the solution was 20mL), to obtain the suspension comprising the radioactive carbon microspheres.

## Comparative Example 4

This comparative example provides a suspension comprising radioactive carbon microspheres, prepared through the following steps:

(1) 10 g of polystyrene/divinylbenzene microspheres (30 $\mu$m) and 120g of dichloroethane were weighed and added with 4.4 g of chloroacetyl chloride and 2 g anhydrous aluminum chloride. The mixture was allowed to react at 30 °C for 24 h, followed by washing sequentially with ethanol and deionized water and drying at 90 °C to obtain polymer microspheres bearing chloroacetyl functional group on the surface thereof;
(2) 5g of microspheres bearing chloroacetyl functional group in step (1) were weighed, and added with 10% sodium hydroxide solution, followed by refluxing for 12h, washing with water for 3 times, and drying at 90°C for use;
(3) 5 g of the microspheres from step (2) were weighed and added to 100 mL of deionized water for mixing with stirring at 60 °C. The mixture was deoxygenated by purging with nitrogen for 1 h. 10 mL of a 0.1 M ceric ammonium nitrate solution (dissolved in 1 M HNO$_3$) was added. After 30 min, 10 mL of glycidyl methacrylate (GMA) was introduced for reaction for 6 h. Upon the reaction was complete, the product was repeatedly washed with deionized water to yield polymer microspheres surface-grafted with GMA, denoted as Poly-g-GMA.
(4) 1 .6 g of iminodiacetic acid (IDA) and 1 g of NaOH were added to 50 mL of water to generate the sodium salt of IDA, then 1 g of Poly-g-GMA microspheres and 2 M Na$_2$CO$_3$ solution were added to adjust the pH to 10-11, and the mixed solution was stirred at 60 °C for 12h. Upon the reaction was complete, the particles were washed repeatedly with deionized water, and the product was denoted as Poly-g-GMA-IDA.
(5) 20 g of 1M yttrium [$^{90}$Y] chloride solution and Poly-g-GMA-IDA microspheres were weighed for sufficient mixing and uniformly mixed by stirring at 40-50 °C for 40-80 minutes. Upon the reaction was complete, the resulting product was washed with saline water repeatedly 5 times. 2g of the microspheres were resuspended in 10mL of water for injection, to yield the suspension comprising yttrium-90 radioactive resin microspheres.

## II. Effect Test Examples

## Test Example 1

Determination of the non-spherical ratio, particle size, and particle size distribution of radioactive microspheres.

(1) Non-spherical ratio: the morphology of the radioactive microspheres was observed by microscopy, and the non-spherical ratio was calculated according to the following procedure:

Measurement method: An appropriate number of radioactive microspheres from the suspensions prepared in Examples 1-10 and Comparative Examples 1-4 was placed onto a disposable hemocytometer. The sample was covered with the cover slip, ensuring the grid face was oriented downward (with the lettering facing upward), and was examined under a trinocular microscope. Four suitable and equally sized square grid areas were randomly selected on the disposable hemocytometer. Observation and photography were performed at a magnification of $20\times$ to$40\times$. The number of non-spherical microspheres and the total number of microspheres (including both spherical and non-spherical microspheres) within the selected four grid areas were counted.

Calculation method: Non-spherical ratio = the number of non-spherical microspheres/total number of microspheres $\times$ 100% (the final result is taken as the average of four areas).

Judgment Criteria: Non-spherical microspheres are defined as irregular particles with a minimum radius of less than 75% of the radius of the initial microspheres, but excluding concave particles, as detailed in FIG. 2, which shows actual microscopic observations of the embodiment comprising non-spherical microspheres, where the painted boxes represent non-spherical microspheres. The final measured results for the non-spherical ratio are shown in Table 1 below.

(2) Particle size and particle size distribution: The measurement method is the same as for the Non-Sphericity Ratio. Utilizing a disposable counting chamber and a trinocular microscope, the microspheres were observed and photographed. Particle size was calculated. Based on the results, the number of particles within the size range of 20 $\mu$m to 60 $\mu$m and the total number of particles within the selected four grid areas were counted.

Calculation method: Percentage of microparticles in the range of 20 $\mu$m to 60 $\mu$m = the number of microparticles in the range of 20 $\mu$m to 60 $\mu$m/total number of microparticles $\times$ 100% (the final result is taken as the average of the four areas).

Criteria: Both non-spherical microspheres and spherical particles were included in the counting, and the particle size of non-spherical microspheres was calculated based on the straight-line distance between the two farthest endpoints on the microsphere.

(3) Mean particle size: The mean particle size of the radioactive microspheres was measured using a Malvern laser particle sizer (Mastersizer 3000).

All of the above tests were performed under conditions after the radioactive microspheres were placed to complete their decay.

The average particle size of the radioactive polystyrene microspheres and carbon microspheres provided in each of the examples and comparative examples were in the range of 25 to 40 $\mu$m, and the percentage of the radioactive microspheres in the range of 20 $\mu$m to 60 $\mu$m was greater than 85%, and the activity range of yttrium [$^{90}$Y] per mL at the time of calibration was in the range of 500 to 700 MBq, and was not significantly different.

Table 1

| No. | Microsphere types | Non-spherical ratio |
|---|---|---|
| Example 1 | Sulfonated polystyrene microspheres | 1.4% |
| Example 2 | Sulfonated polystyrene microspheres | 5.0% |
| Example 3 | Sulfonated polystyrene microspheres | 7.2% |
| Example 4 | Sulfonated polystyrene microspheres | 4.6% |
| Example 5 | Sulfonated polystyrene microspheres | 2.2% |
| Example 6 | Carbon microspheres | 1.5% |
| Example 7 | Carbon microspheres | 4.8% |
| Example 8 | Carbon microspheres | 9.4% |
| Example 9 | Carbon microspheres | 4.5% |
| Example 10 | Carbon microspheres | 3.4% |
| Comparative Example 1 | Sulfonated polystyrene microspheres | 15.2% |
| Comparative Example 2 | Sulfonated polystyrene microspheres | 8.0% |
| Comparative Example 3 | Carbon microspheres | 15.1% |

(continued)

| No. | Microsphere types | Non-spherical ratio |
|---|---|---|
| Comparative Example 4 | Poly-g-GMA-IDA microspheres | 12.4% |

According to Table 1, the non-spherical ratios of both the radioactive polystyrene microspheres and the radioactive carbon microspheres of Examples 1 to 10 can be controlled to 10% or less by utilizing the method provided in the present application. Among them, the non-spherical ratios of Examples 1 to 2, 4 to 7, and 9 to 10 can all be controlled to 5% or less.

**Test Example 2**

Determine and evaluate whether radioactive microspheres lodged in the tumor microcirculation elicit irritation in mammals and assess the severity level.

Test samples: the radioactive microspheres from Examples 1-3, Examples 6-8, and Comparative Examples 1 and 3.

Control: high-density polyethylene film (HDPE) (Hatano Research Institute, FDSC, Lot C-212).

Experimental animals: experimental New Zealand white rabbits, general grade, male; Weight: 2.5-3.5 kg.

Experimental Methods:

(1) The white rabbits were divided into 8 groups of 3 rabbits each, and the 8 groups corresponded to Examples 1-3, Examples 6-8, and Comparative Example 1 and 3, respectively. Within 24 hours prior to the experiment, hair was removed from both lateral sides of the animal's dorsum. The anesthetized animals were placed in a prone position on the operating table, and isoflurane anesthesia was used and maintained during the procedure to prevent muscle twitching in the animals.

(2) After the test samples were left to decay, the suspension was centrifuged to remove the liquid phase.

(3) A longitudinal incision was made along the dorsal skin midline of the animal. After dissecting the fascia, the paraspinal muscles were exposed. A small incision was created in the muscle using a scalpel, and a quantity of processed test samples or controls (approximately 0.5 g of resin microspheres or approximately 0.3 g of carbon microspheres) was implanted into the incision sites as illustrated in FIG. 3 using hemostatic forceps. Four test samples (T1, T2, T3, T4) were implanted into the left paraspinal muscles of each animal, while four controls (C1, C2, C3, C4) were implanted into the contralateral corresponding muscles.

(4) The day of implantation was defined as Day 1. All implants were placed about 25 mm-50 mm away from the midline and parallel to the midline, with a spacing of about 2.5 cm between each implant, and the muscle and skin were sutured using medical sutures after implantation was completed. Aseptic technique was ensured throughout the implantation procedure, and the implantation period was set at 13 weeks.

(5) Following the implantation period, animals were euthanized. A longitudinal incision was made along the dorsal center of the rabbit to expose the paraspinal muscles, from which sufficiently large sections of the paraspinal muscles encapsulating the implants and unaffected by surgical intervention were excised. All implantation sites were collected and fixed and then subjected to visual observation and histopathological evaluation.

Evaluation Criteria:

The experimental results were evaluated mainly based on the histopathological examination results, and the specific grading process was as follows:

(1) Both cellular and tissue responses in the local tissues surrounding each implant were evaluated and recorded.

(2) The cellular response score (scored in Table 2) and the tissue response score (scored in Table 3) were calculated separately for each implantation site.

(3) The total score for each site was calculated respectively as: Total Cellular Response Score $\times$ 2 + Total Tissue Response Score.

(4) Average score per animal test sample = total score for that animal test sample/number of validly implanted test samples. The overall mean score for test samples was calculated by summing the average scores of test samples from each animal and dividing by the number of animals. The overall mean score for controls was calculated in the same manner.

(5) Final Response Score = Total Average Score for the test-samples - Total Average Score for the controls (negative numbers are recorded as 0).

Based on the final response scores, the degree of implantation response for test samples was graded according to Table 4.

Table 2 Histologic evaluation system-cell type/response

| Cell type/response | Score | | | | |
|---|---|---|---|---|---|
| | 0 | 1 | 2 | 3 | 4 |
| Polymorphonuclear leukocyte | 0 | Rare, 1~5phf | 5~10 phf | Heavy infiltration | Full field of view |
| Lymphocyte | 0 | Rare, 1~5phf | 5~10 phf | Heavy infiltration | Full field of view |
| Plasmacyte | 0 | Rare, 1~5phf | 5~10 phf | Heavy infiltration | Full field of view |
| Macrophage | 0 | Rare, 1~5phf | 5~10 phf | Heavy infiltration | Full field of view |
| Giant cell | 0 | Rare, 1~2phf | 3~5 phf | Heavy infiltration | Patchy distribution |
| Necrotic | 0 | Rare | Mild | Moderate | Severe |
| phf-high magnification (400×) field of view | | | | | |

Table3 Histological evaluation system-tissue response

| Type of tissue response | Score | | | | |
|---|---|---|---|---|---|
| | 0 | 1 | 2 | 3 | 4 |
| Neovascularization | 0 | Mild capillary hyperplasia, focal, 1 to 3 buds | 4-7 groups of capillary proliferation accompanied by fibroblastic components | Large-scale capillary proliferation accompanied by fibroblastic components | Extensive capillary proliferation accompanied by fibroblastic components |
| Fibrillation | 0 | Restricted area | Medium-thickness area | Thick area | Extensive area |
| Fatty infiltration | 0 | Rare adipocytes accompanied by fibrosis | Several layers of adipocytes and fibrosis | Extension and enlargement of the area of adipocyte aggregation at the implantation site | The implant is completely surrounded by adipocytes |

Table 4 Grading scale for degree of local response after implantation

| Final Response Score | Grade |
|---|---|
| 0-2.9 | No or very mild irritation |
| 3.0- 8.9 | Mild irritation |
| 9.0-15.0 | Moderate irritation |
| >15.0 | Severe irritation |

The results of the experiment are as follows:

No dying or dead animals were found during the test. Planned dissections were performed and no test sample-related gross changes were seen.

The final response scores of the test samples compared to the controls are shown in Table 5 below.

Table 5. Results of implantation experiments

| Groups | Example 1 | Example 2 | Example 3 | Example 6 | Example 7 | Example 8 | Comparative Example 1 | Comparative Example 3 |
|---|---|---|---|---|---|---|---|---|
| Score | 0.8 | 1.2 | 3.8 | 1.1 | 1.7 | 4.3 | 8.5 | 9.1 |

As can be seen in Table 5, at 13 weeks of implantation, Examples 1, 2, 6, and 7 exhibited low scores of 2.9 or less, while Examples 3 and 8 had slightly higher scores, and Comparative Examples 1 and 3 had the highest score of nearly 9.0. This indicates that when the non-spherical ratio of radioactive microspheres in the suspension is 5% or less, the suspension is rated as having no irritation. In contrast, a non-spherical ratios of greater than 5% results in more pronounced irritation or even moderate irritation. Thus, controlling the non-spherical ratio of radioactive microspheres in the suspension to 5% or less significantly reduces local inflammatory response and improves safety.

**Test Example 3**

Determination and evaluation of microsphere distribution in rabbit liver tumors.
Test samples: the radioactive microspheres from Examples 1-3, Examples 6-8, and Comparative Examples 1 and 3.
Experimental animals: experimental New Zealand white rabbits, general grade, male; Weight: 2.5-3.5 kg.

Experimental Methods:

(1) Tumor model was established.

VX2 rabbit implanted hepatic carcinoma models were established in New Zealand White rabbits by implanting 2-mm diameter VX2 tumor mass into the subcapsular region of the median liver lobe. Postoperative day 7, CT scans were performed to record the location and size of the tumor mass. Tumor size with a maximum diameter of 1-2 cm indicates successful establishment of the tumor model.

(2) Drug administration in groups

Animals were anesthetized preoperatively (2.5% sodium pentobarbital, 30 mg/kg) before operation. The animal's groin was shaved; the skin of the groin was incised; and the femoral artery is bluntly dissected; the femoral artery was punctured under direct vision and a 5F arterial sheath was inserted; both the femoral artery and arterial sheath were secured with medical absorbable sutures. Under the guidance of digital subtraction angiography (DSA), a 2.7F microcatheter was inserted into the common hepatic artery; radioactive microspheres were injected through the microcatheter, with active prevention of microsphere reflux. Following embolization completion, the catheter and arterial sheath were withdrawn; the femoral artery was ligated above the puncture point; muscle and skin were sutured in layers.
The rabbits were randomly divided into 8 groups of 3 rabbits each, and the 8 groups corresponded to Examples 1~3, Examples 6~8, and comparative example 1 and 3, respectively. Following complete decay of the 1 GBq test sample, it is administered into the liver. After 24h, the animal was euthanized and subjected to necropsy; the liver was fixed in formalin, embedded in paraffin and sectioned, and stained with hematoxylin and eosin for pathological examination; microsphere distribution and density are calculated according to the following method.
The area under observation was divided into 4 sections, which were:
0-1 mm external to the tumor border, 0-1 mm internal to the tumor border, non-tumorous tissue, and deep tumor region (>1 mm internal to the tumor border).
Within the field of view of the microscope (3 mm × 3 mm), the distribution density of radioactive microspheres (unit: grains/mm$^2$) was calculated for each region. The average distribution density of the corresponding region for each test sample was calculated according to the following formula:

Mean distribution density = total distribution density/number of animals in the corresponding region.

The results of the experiment are shown in Table 6 below.

Table 6

| groups | Average distribution density | | | |
|---|---|---|---|---|
| | 0-1 mm external to the tumor border | 0-1 mm internal to the tumor border | non-tumorous tissue | deep tumor region |
| Example 1 | 2.1 | 3.1 | 0.1 | 1.8 |
| Example 2 | 2.0 | 3.2 | 0.4 | 2.0 |
| Example 3 | 2.5 | 3.3 | 0.8 | 1.2 |
| Example 6 | 2.2 | 3.2 | 0 | 1.6 |
| Example 7 | 2.4 | 3.5 | 0.1 | 1.7 |
| Example 8 | 2.6 | 2.8 | 0.7 | 1.3 |
| Comparative Example 1 | 2.9 | 3.1 | 1.4 | 0.5 |
| Comparative Example 3 | 2.7 | 2.9 | 1.3 | 0.3 |

As can be seen from Table 6, comparative to Comparative Examples 1 and 3, Example 1, Example 2, Example 6, and Example 7 overall have a higher average distribution density within the tumor, and in particular have a significantly higher average distribution density in the deep tumor region, and there is a decrease in the density of extra tumoral and non-tumoral tissues, e.g., the non-tumoral tissues in Example 1 have a distribution density of 0.1 wile 1.4 for comparative example 1, and Example 1 have a distribution density of 1.8 in the deep tumor region while 0.5 for Comparative example 1. It is shown that controlling the non-spherical ratio of radioactive microspheres in the suspension to 5% or less can enhance the intratumoral distribution density while decreasing the distribution density externally to the tumor border.

**Test Example 4**

Determination and evaluation of therapeutic effects of radioactive microsphere on rabbit liver tumors.
Test samples: the radioactive microspheres from Examples 2, 7, and Comparative Examples 1 and 3.
Experimental animals: experimental New Zealand white rabbits, general grade, male; Weight: 2.5~3.5kg.

Experimental Methods:

(1) Tumor model was established.

VX2 rabbit implanted hepatic carcinoma models were established in New Zealand White rabbits by implanting 2-mm diameter VX2 tumor mass into the subcapsular region of the median liver lobe. Postoperative after, CT scans were performed to record the location and size of the tumor mass. Tumors size up to a maximum diameter of 1-2 cm is considered successful for modeling.

(2) Drug administration in groups

The rabbits were randomly divided into 5 groups of 3 rabbits each, and the 5 groups corresponded to Examples 2 and 7, comparative example 1 and 3, and model, respectively. Among them, Examples 2 and 7, and Comparative Examples 1 and 3 were classified into the treatment group.
Model group: animals were anesthetized (2.5% sodium pentobarbital, 30 mg/kg) before operation. The animal's groin was shaved; the skin of the groin was incised; and the femoral artery is bluntly dissected; the femoral artery was punctured under direct vision and a 5F arterial sheath was inserted; both the femoral artery and arterial sheath were secured with medical absorbable sutures. Under the guidance of digital subtraction angiography (DSA), a 2.7F microcatheter was inserted into the common hepatic artery; saline was injected through the microcatheter, with active prevention of microsphere reflux. Following embolization completion, the catheter and arterial sheath were withdrawn; the femoral artery was ligated above the puncture point; muscle and skin were sutured in layers.
A catheter was inserted into the rabbit's common hepatic artery and saline was slowly injected through the catheter.
Treatment group: each was given radioembolization microspheres through surgical intervention at a dose of 1 GBq, and the intervention method was the same as that of the model group.
After 2 weeks of drug administration, CT scans were performed to record the location and size of the tumor, and the animals were dissected, stripped of the tumor, and weighed. The tumor growth inhibition rate was calculated, and the results are shown in Table 7.

The tumor growth inhibition rate was calculated according to the following formula:

Tumor growth inhibition rate = (average tumor weight of animals in the mod-1 group - average tumor weight of animals in the treatment group)/average tumor weight of animals in the model group × 100%.

Table 7

| Groups | Example 2 | Example 7 | Comparative Example 1 | Comparative Example 3 |
|---|---|---|---|---|
| Tumor growth inhibition rate | 85.0% | 81.0% | 61.6% | 58.0% |

As can be seen from Table 7, compared to Comparative Examples 1 and 3, Example 2 and Example 7 overall have a higher tumor growth inhibition rate for liver tumors. For example, the tumor growth inhibition rate of Example 2 is 85.0%, while the tumor growth inhibition rate of comparative example 1 is only 61.6%. Both Example 2 and Example 7 achieve an increase of 20% or more in tumor growth inhibition rate. It was shown that controlling the non-spherical ratio to 5% or less has a better tumor suppression effect, as it enhances the distribution density of microspheres within the tumor and thus enhances the tumor killing ability.

Although the present invention has been described in detail above with general description, specific embodiments and tests, some modifications or improvements can be made on the basis of the present invention, as will be obvious to those skilled in the art. Therefore, these modifications or improvements made without departing from the spirit of the present disclosure fall within the scope of the claimed protection of the present invention.

**Claims**

1.    A suspension, wherein the suspension comprises radioactive microspheres, and the radioactive microspheres comprise resin microspheres and radionuclides loaded on the resin microspheres, the radioactive microspheres have an average particle size of 25 μm to 40 μm, and the radioactive microspheres have a non-spherical ratio of 5% or less.

2.    The suspension according to claim 1, wherein the non-spherical ratio means the ratio of the number of non-spherical radioactive microspheres to the total number of radioactive microspheres in the suspension, and a non-spherical radioactive microsphere is a radioactive microsphere with a minimum radius of less than 75% of the radius of an initial microsphere;

and/or, in the suspension, the radioactive microspheres have a non-spherical ratio or less;
and/or, in the suspension, the percentage of radioactive microspheres having a particle size of 20 μm to 60 μm is greater than 85% in terms of the number of the radioactive microspheres;
and/or, the material of the resin microspheres is selected from at least one of crosslinked polystyrene resin, crosslinked polyethylene resin and crosslinked polydivinylbenzene resin, preferably, the material of the resin microspheres is selected from a polystyrene resin carrying sulfonic acid groups and crosslinked with divinylbenzene;
and/or, the resin microspheres are any one of resin microspheres carrying sulfonic acid groups, resin microspheres carrying nitro groups, and carbonized resin microspheres;
and/or, the radionuclides are selected from an isotope of at least one of yttrium, lutetium, indium, holmium, samarium, iodine, phosphorus, iridium, or rhenium, preferably the radionuclides are selected from yttrium [90Y];
and/or, the suspension further comprises water for injection.

3.    The suspension according to claim 1 or 2, wherein each mL of the suspension comprises yttrium [90Y] with an activity of 300-700 MBq.

4.    A suspension comprising radioactive microspheres, wherein the radioactive microspheres comprise resin microspheres and radionuclides loaded on the resin microspheres, the material of the resin microspheres comprises at least one of polystyrene, polyethylene, or polydivinylbenzene, or a crosslinked polymer thereof, and the radioactive microspheres have an average particle size of 25 μm to 40 μm, and have a non-spherical ratio of 5% or less.

5. The suspension according to claim 4, wherein the non-spherical ratio means the ratio of the number of non-spherical radioactive microspheres to the total number of radioactive microspheres in the suspension, and a non-spherical radioactive microsphere is a radioactive microsphere with a minimum radius of less than 75% of the radius of an initial microsphere.

6. The suspension according to claim 4 or 5, wherein in the suspension, the percentage of radioactive microspheres having a particle size of 20 $\mu$m to 60 $\mu$m is greater than 85% in terms of the number of the radioactive microspheres; and/or the radioactive microspheres have a non-spherical ratio or less; and/or, the material of the resin microspheres is selected from crosslinked polystyrene microspheres with a crosslinking degree to 10%.

7. The suspension comprising radioactive microspheres according to at least one of claims 4-6, **characterized in that** the resin microspheres are sulfonated, nitrated or carbonized resin microspheres.

8. The suspension comprising radioactive microspheres according to at least one of claims 4-7, **characterized in that**, the radionuclides are selected from an isotope of at least one of yttrium, lutetium, indium, holmium, samarium, iodine, phosphorus, iridium, or rhenium.

9. The suspension comprising radioactive microspheres according to at least one of claims 4-8, wherein the material of the resin microspheres is selected from a polystyrene resin carrying sulfonic acid groups and crosslinked with divinylbenzene.

10. The suspension comprising radioactive microspheres according to at least one of claims 4-9, **characterized in that** the suspension further comprises water for injection.

11. The suspension comprising radioactive microspheres according to at least one of claims 4-10, wherein the radionuclides are selected from yttrium [$^{90}$Y], and each mL of the suspension comprises yttrium [$^{90}$Y] with an activity of 300-700 MBq.

12. The suspension comprising radioactive microspheres according to at least one of claims 4-11, **characterized in that** the radionuclides are selected from yttrium [$^{90}$Y], the material of the resin microspheres is selected from sulfonated polystyrene partially crosslinked with divinylbenzene, the suspension further comprises water for injection, and each mL of the suspension comprises yttrium [$^{90}$Y] with an activity of 300-700 MBq.

13. A preparation method for a suspension comprising radioactive microspheres, **characterized in that**, the method comprises: mixing resin microspheres with a radionuclide compound in a solution to react, thereby obtaining the suspension comprising radioactive microspheres;
wherein the material of the resin microspheres comprises at least one of polystyrene, polyethylene, or polydivinylbenzene, or a crosslinked polymer thereof, and the radioactive microspheres have an average particle size of from 25 $\mu$m to 40 $\mu$m, and a non-spherical ratio of 5% or less.

14. The preparation method according to claim 13, **characterized in that** the resin microspheres are subjected to sulfonation, nitration or carbonization treatment prior to being mixed with the radionuclide compound.

15. A preparation method for a suspension, comprising:

    mixing the resin microspheres and a radionuclide compound in a solution for reacting to obtain the suspension comprising radioactive microspheres;
    wherein in the suspension, the radioactive microspheres have an average particle size of 25 $\mu$m to 40 $\mu$m and a non-spherical ratio of 5% less.

16. The preparation method according to claim 15, wherein the resin microspheres are subjected to sulfonation, nitration or carbonization treatment prior to being mixed with the radionuclide compound.

17. Use of The suspension according to any one of claims 1 to 12, or the suspension prepared by the method according to any one of claims 13-16 in the preparation of a medicament for treating a tumor, wherein the tumor is preferably a primary or secondary liver cancer.

18. A method of treating a tumor, comprising:

# EP 4 740 968 A1

administering to a tumor patient a suspension, wherein the suspension comprises radioactive microspheres, and the radioactive microspheres comprise resin microspheres and radionuclides loaded on the resin microspheres, the radioactive microspheres have an average particle size of 25 $\mu$m to 40 $\mu$m, and the radioactive microspheres have a non-spherical ratio of 5% or less.

19. The method according to claim 18, wherein the tumor is primary or secondary liver cancer.

20. The method according to claim 18 or 19, wherein the non-spherical ratio means the ratio of the number of non-spherical radioactive microspheres to the total number of radioactive microspheres in the suspension, and a non-spherical radioactive microsphere is a radioactive microsphere with a minimum radius of less than 75% of the radius of an initial microsphere;

and/or, the material of the resin microspheres is selected from at least one of crosslinked polystyrene resin, crosslinked polyethylene resin and crosslinked polydivinylbenzene resin, preferably the material of the resin microspheres is selected from a polystyrene resin carrying sulfonic acid groups and crosslinked with divinylbenzene;
and/or, the resin microspheres are any one of resin microspheres carrying sulfonic acid groups, resin microspheres carrying nitro groups, and carbonized resin microspheres;
and/or, the radionuclides are selected from an isotope of at least one of yttrium, lutetium, indium, holmium, samarium, iodine, phosphorus, iridium, or rhenium, preferably the radionuclides are selected from yttrium [$^{90}$Y], and each mL of the suspension comprises yttrium [$^{90}$Y] with an activity of 300-700 MBq.

$R_{min}=R$ √    $R_{min}=0<0.75R$ ×    $R_{min}<0.75R$ ×    $R=0.71R<0.75R$ ×

**FIG. 1**

**FIG. 2**

head

Left
Test sample
implantation sites

Right
Control implantation
sites

Tail

**FIG. 3**

## INTERNATIONAL SEARCH REPORT

| International application No. |
|---|
| **PCT/CN2024/103920** |

**A. CLASSIFICATION OF SUBJECT MATTER**

A61K51/12(2006.01)i; A61K51/06(2006.01)i; A61K103/32(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS, DWPI, WPABS, CNTXT, ENTXT, WOTXT, USTXT, EPTXT, JPTXT, CNKI, PubMed, ISI Web of Knowledg: 成都社泰医疗科技有限公司, 放射, 微球, 树脂微球, 粒径, 非球形, 球形, 聚苯乙烯, 聚乙烯, 聚二乙烯苯, 磺酸基, 磺化, 硝基, 硝化, 炭化, 钇, 肿瘤, 肝癌, radioactive, microsphere, resin microsphere, polystyrene, polyethylene, polydivinylbenzene, sulfonated, yttrium, tumor, liver cancer

**C. DOCUMENTS CONSIDERED TO BE RELEVANT**

| Category* | Citation of document, with indication, where appropriate, of the relevant passages | Relevant to claim No. |
|---|---|---|
| PX | CN 116549679 A (BEIJING PUER WEIYE BIOTECHNOLOGY CO., LTD.) 08 August 2023 (2023-08-08)<br>claims 1-10 | 1-20 |
| PX | CN 117257996 A (BEIJING PUER WEIYE BIOTECHNOLOGY CO., LTD.) 22 December 2023 (2023-12-22)<br>claims 1-10 | 1-20 |
| X | WO 0234300 A1 (SIRTEX MEDICAL LTD.) 02 May 2002 (2002-05-02)<br>claims 1-13 and 18-23, and description, page 6, lines 18-20, and embodiment 1 | 1-11, 13-20 |
| Y | WO 0234300 A1 (SIRTEX MEDICAL LTD.) 02 May 2002 (2002-05-02)<br>claims 1-13 and 18-23, and description, page 6, lines 18-20, and embodiment 1 | 2-3, 7-12, 14, 16, 17, 20 |
| Y | CN 113845610 A (XI'AN NARUI INDUSTRIAL TECHNOLOGY CO., LTD.) 28 December 2021 (2021-12-28)<br>description, paragraphs 0005 and 0029 | 2-3, 7-12, 14, 16, 17, 20 |

✓ Further documents are listed in the continuation of Box C.　　✓ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **30 September 2024** | **10 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

# EP 4 740 968 A1

**INTERNATIONAL SEARCH REPORT**

| International application No. |
| --- |
| **PCT/CN2024/103920** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 115607694 A (BEIJING PUER WEIYE BIOLOGICAL TECHNOLOGY CO., LTD.) 17 January 2023 (2023-01-17)<br>description, paragraphs 0005 and 0029 | 2-3, 7-8, 10-11, 14, 16-17, 20 |
| A | CN 115282298 A (SUZHOU KNOWLEDGE & BENEFIT SPHERE TECHNOLOGY CO., LTD.) 04 November 2022 (2022-11-04)<br>abstract, and description, paragraph 0003, and embodiment 1 | 1-20 |
| A | CN 102671220 A (CHENGDU YUNKE PHARMACEUTICAL CO., LTD.) 19 September 2012 (2012-09-19)<br>entire document | 1-20 |
| A | CN 105451780 A (THE AUSTRALIAN NATIONAL UNIVERSITY) 30 March 2016 (2016-03-30)<br>entire document | 1-20 |

Form PCT/ISA/210 (second sheet) (July 2022)

22

**INTERNATIONAL SEARCH REPORT**

| International application No. |
| --- |
| **PCT/CN2024/103920** |

| Box No. II      Observations where certain claims were found unsearchable (Continuation of item 2 of first sheet) |
| --- |

This international search report has not been established in respect of certain claims under Article 17(2)(a) for the following reasons:

1. ☑   Claims Nos.: **18-20**
   because they relate to subject matter not required to be searched by this Authority, namely:

   Claims 18-20 set forth a method for treating tumors, and no international search can be performed on such subject matter according to the criteria set out in PCT Rule 39.1(iv). The search report is provided on the basis that the subject matter of claims 18-20 is "the use of a suspension in the preparation of a drug for treating tumors".

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| | International application No. |
|---|---|
| | **PCT/CN2024/103920** |

| Patent document cited in search report | | | Publication date (day/month/year) | Patent family member(s) | | | Publication date (day/month/year) |
|---|---|---|---|---|---|---|---|
| CN | 116549679 | A | 08 August 2023 | None | | | |
| CN | 117257996 | A | 22 December 2023 | None | | | |
| WO | 0234300 | A1 | 02 May 2002 | AUPR | 098300 | A0 | 16 November 2000 |
| | | | | AU | 1027802 | A | 06 May 2002 |
| | | | | US | 2014099255 | A1 | 10 April 2014 |
| | | | | US | 2010215571 | A1 | 26 August 2010 |
| | | | | JP | 2004511576 | A | 15 April 2004 |
| | | | | JP | 4229699 | B2 | 25 February 2009 |
| | | | | US | 2003007928 | A1 | 09 January 2003 |
| | | | | US | 2007253898 | A1 | 01 November 2007 |
| | | | | CA | 2426602 | A1 | 02 May 2002 |
| | | | | CA | 2426602 | C | 25 October 2022 |
| | | | | US | 2019175768 | A1 | 13 June 2019 |
| | | | | US | 11097021 | B2 | 24 August 2021 |
| | | | | EP | 1333866 | A1 | 13 August 2003 |
| | | | | EP | 1333866 | A4 | 06 June 2007 |
| CN | 113845610 | A | 28 December 2021 | None | | | |
| CN | 115607694 | A | 17 January 2023 | None | | | |
| CN | 115282298 | A | 04 November 2022 | None | | | |
| CN | 102671220 | A | 19 September 2012 | None | | | |
| CN | 105451780 | A | 30 March 2016 | AU | 2013393811 | A1 | 21 January 2016 |
| | | | | AU | 2013393811 | B2 | 08 November 2018 |
| | | | | TW | 201536331 | A | 01 October 2015 |
| | | | | BR | 112015032632 | A2 | 25 July 2017 |
| | | | | IL | 243361 | B | 28 February 2018 |
| | | | | NZ | 631075 | A | 30 June 2017 |
| | | | | CA | 2915880 | A1 | 08 January 2015 |
| | | | | CA | 2915880 | C | 23 July 2019 |
| | | | | WO | 2015000012 | A1 | 08 January 2015 |
| | | | | US | 2016151518 | A1 | 02 June 2016 |
| | | | | US | 10232062 | B2 | 19 March 2019 |
| | | | | EP | 3016688 | A1 | 11 May 2016 |
| | | | | EP | 3016688 | A4 | 08 February 2017 |
| | | | | EP | 3016688 | B1 | 25 April 2018 |
| | | | | HK | 1220906 | A1 | 19 May 2017 |
| | | | | JP | 2016525079 | A | 22 August 2016 |
| | | | | JP | 6461124 | B2 | 30 January 2019 |
| | | | | KR | 20160029077 | A | 14 March 2016 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2023108208986
- CN 202211592412X
- CN 2022116010239
- CN 2022116017505